# EUROPEAN PATENT SPECIFICATION

(11) **EP 1 494 686 B1**
(45) Date of publication and mention of the grant of the patent: **30.06.2010**
(21) Application number: 03719708.4
(22) Date of filing: 08.04.2003
(51) Int. Cl.: A61K 31/715

(54) **ORAL REHYDRATION COMPOSITION**
ORALE REHYDRIERUNGSZUSAMMENSETZUNG
COMPOSITION DE REHYDRATATION ORALE

(30) Priority: 16.04.2002 US 125367
(43) Date of publication of application: 12.01.2005
(73) Proprietor: Cera Products, Inc., Jessup, MD 20794 (US)
(72) Inventor: MITCHELL, Cheryl, Stockton, CA 98219 (US); RIIKONEN, Charlene, Columbia, MD 21044 (US); SACK, David, A., Fallston, MD 21047 (US)
(74) Representative: Doble, Richard George Vivian
(86) International application number: PCT/US2003/011226
(87) International publication number: WO 2003/088901

(56) References cited:
- GB-A- 2 363 073
- US-A- 5 096 894
- US-A- 5 096 894
- US-A- 5 489 440
- US-A- 5 489 440
- US-A- 6 120 814

## Description

### FIELD OF THE INVENTION

The present invention relates to the field of rehydration compositions. More specifically, the present invention relates to dried, or dehydrated, rehydration compositions comprising, at the least, a low fiber, colloidal, hydrolyzed, rice based carbohydrate ingredient.

### BACKGROUND

Historically, in cases of dehydration caused by excessive sweating or illness resulting in body fluid loss, replenishment of lost body fluids by water is essential. While water is an essential component in fluid replacement, it is also recognized that certain salts containing ions (electrolytes) such as sodium, potassium, and citrate must be replaced along with the water. In general, aqueous solutions containing just these salts are not well absorbed by the body and are not organoleptically acceptable. That is, most people find aqueous salt solutions very difficult to consume.

More recently, it was discovered that carbohydrates, more specifically glucose, promote the absorption of these ions as well as providing sufficient sweetness to promote organoleptic acceptability of the product. During the last forty years, the World Health Organization has promoted an oral rehydration composition that utilizes glucose in combination with electrolytes. This composition, when dissolved in water, produces an oral rehydration solution "ORS", which has had a significant impact on the survival rate of cholera victims. It is known that different carbohydrate sources have been utilized in an effort to improve the absorption of the electrolytes.

### PRIOR ART

We have previously disclosed that when using a rice carbohydrate base (prepared by the method of Mitchell U.S. Patent No. 4,876,096 and 4,756,912), having a dextrose equivalency (DE) of 35 to 50, and a glucose and maltose content of between 20 and 40% dry substance basis, that we were able to use 40 grams/liter of carbohydrates rather than 20 grams/liter of carbohydrates as glucose as provided in the WHO standard glucose ORS. The glucose content in non-rice carbohydrate base based ORS cannot be increased above 20 grams/liter without increasing osmolarity beyond the limiting point of 300 milliosmoles, which would result in diarrhea and an unwanted increase in fluid losses. By using 40 grams/liter of rice based carbohydrates in the ORS, we were able to provide significantly more carbohydrate content while maintaining an osmolarity of less than 300 milliosmoles. The increase in rice-based carbohydrates in our prior art oral rehydration compositions, and subsequent solutions, promotes an increased uptake or absorption of the electrolytes over the standard glucose based ORS by 20 to 30% (Cera's cholera study, published by A. Paediatricia in June 2001). Other products on the market use a DE 30 rice based carbohydrate, which has been highly filtered and refined so as to be clear. These rice-based carbohydrate ORS formulations do not contain the non-carbohydrate components fat and protein. Further, there is a European product (not sold in the US) that combines glucose with ground rice. Notably, all oral rehydration compositions have a significant content of simple sugars such as glucose, fructose, or maltose for the purpose of improving organoleptic acceptance, and, more importantly, for the perceived notion that these simple sugars are essential in oral rehydration solutions because of their carrying power that enables the metabolic absorption of the ions.

Some prior art teaches the use of a form of rice flour in the preparation of oral rehydration compositions. U.S. Patent 5,096,894, by Tao et al., for example, specifies the absolute need for a clarified rice dextrin in the preparation of rice based oral rehydration compositions and solutions. Tao stated that rice dextrins having greater than 0.1 % protein are "not suitable as the carbohydrate component of the instant ORS due to trace amounts of particulate matter and residual protein which contributes to foaming and browning problems during processing and sterilization and the formation of fine precipitate during storage". The claims of Tao et al. reflect this issue by claiming an ORS based on a rice dextrin having 50 to 90% short chains of 2 to 6 carbons and having less than 0.1 % protein.

U.S. Patent 5,489,440, by Ndife et al., teaches the manufacture of an oral rehydration composition and solution using a gelatinized rice starch and not a rice dextrin. Food grade dextrins are produced by the action of alpha-amylase or acids on the gelatinized rice starch. Ndife teaches the modification of rice flour using only protease and cellulase to solubilize the fibers and proteins. The latter process yields a gelatinized starch product having a DE considerably less than 5, which contributes significantly to the viscosity of solution in which it is used as an ingredient.

U.S. Patent 5,120,539, by Lebenthal et al., teaches the preparation of an oral rehydration formula using rice flour that had been hydrolyzed using alpha amylases. The '539 product contains both insoluble, non-enzymatically hydrolyzed, fiber and protein from the rice flour.

### SUMMARY OF THE INVENTION

It is an object of the present invention to provide an oral rehydration composition, comprising electrolytes and carbohydrates, that is suitable for dissolving in a water base so as to produce an oral rehydration solution having an osmolality of less than 400 milliomoles; said carbohydrates further comprising a low fiber colloidal hydrolyzed rice suspension comprising, on a dry weight basis, less than 0.1% fiber, between 0.5% and 1.0% protein, between 0.5% and 1.0% fat, a dextrose equivalency of between 20 and 30, a glucose and maltose content of less than 20%, on a dry weight basis; and an effective amount of electrolytes.

It is another object of the present invention to provide an oral rehydration composition, as above, further comprising an effective amount of nutritive or non-nutritive sweeteners to improve organoleptic qualities.

It is a further object of the present invention to provide an oral rehydration composition, as above, further comprising of at least one nutraceutical or at least one functional ingredient.

The novel features that are considered characteristic of the invention are set forth with particularity in the appended claims. The invention itself, however, both as to its structure and its operation together with the additional objects and advantages thereof will best be understood from the following description of the preferred embodiment of the present invention. Unless specifically noted, it is intended that the words and phrases in the specification and claims be given the ordinary and accustomed meaning to those of ordinary skill in the applicable art or arts. If any other meaning is intended, the specification will specifically state that a special meaning is being applied to a word or phrase.

### DESCRIPTION OF THE PREFERRED EMBODIMENT

We found that when electrolytes, such as sodium, potassium, citrate or bicarbonate, were added to a cloudy rice base, more specifically a low fiber colloidal hydrolyzed rice suspension, even more specifically one that has a very low fiber content of less than 0.1 % fiber, a protein content of between 0.5% and 1.0%, and a fat content of between 0.5% and 1.0%, while also having a carbohydrate content that has been enzymatically hydrolyzed to a DE within the range of 20 to 30, and further having a combined glucose and maltose content of less than 20% on a dry substance basis, we maximized the load of carbohydrates, electrolytes, and functional ingredients in the ORS and still maintain the accepted limits of osmolarity below 300 milliosmoles so as to avoid causing diarrhea. To our surprise, oral rehydration solutions resulting from the use of the maximum load of lower DE cloudy rice base carbohydrates and having relatively small amounts of glucose or maltose, provide an even greater stability, absorption, and carrying power for electrolytes.

Additionally, other ingredient compounds that support the healthy maintenance of the body (generally referred to as "nutraceuticals" or "functional" ingredients) could then be added and carried by the cloudy carbohydrate base. However, because of the poor taste of the product (lack of any sweetness), the organoleptic acceptance of the product made exclusively with carbohydrates having a DE 20 to 30 is extremely limited when compared with products containing glucose, sucrose, or combination of glucose and maltose as contained in grain derived carbohydrate syrups having a DE of 30 to 55. The endogenous simple sugars of the DE 30 to 55 hydrolysates provide sufficient sweetness to make the taste acceptable, but also contribute to the osmolarity. Combinations of flavors, nutritive or non-nutritive sweeteners, or functional or nutraceutical adjunct treatments such as vaccines, amino acids, peptides, complex carbohydrates such as inulin, or mineral salts, which we were unable to add previously with the 40 grams/liter of cloudy DE 30-50 rice base and electrolyte blend, can now be added without increasing the osmolarity beyond the desired limit of 300 milliosmoles per liter. We also recognize that the cloudy starch hydrolysates having a DE of between 20 and 30 provide not only an active means that promote transport of the electrolytes and other ingredients through the metabolic uptake, but additionally provided stability of the dried oral rehydration composition or delivery system containing more sensitive ingredients. In prior art, dried compositions containing starch hydrolysate carbohydrates with the higher DE (35-50) tend to crystallize and harden when subjected to heat greater than 29.4° C to 32.2° C (85° to 90° F). Whereas, we found that the lower DE starch hydrolysate products in the oral rehydration compositions of this invention, do not crystallize until temperatures are 43.3° C (110° F) or hotter. This is an important consideration for shelf life, storage, and use of dried oral rehydration compositions that are distributed in warm climates as the dry package form.

In considering that glucose was not an essential part of an effective oral rehydration composition and, in fact, discovering that cloudy rice starch hydrolysates containing virtually no glucose (less than 10% on a dry substance basis) could be used in an oral rehydration composition to produce an extremely effective ORS, we also discovered that cloudy rice starch hydrolysates that contain essentially no fiber but do contain a protein content of between 0.5% and 1%, and fat content of between 0.5% and 1%, as in the rice oligodextrins or syrups prepared by the method of Mitchell, were even more effective in transporting electrolytes or nutraceuticals and have a superior effect on rehydration and replacement of fluid loss. Also, because of lower osmolarity resulting from solutions containing the DE 20-30 carbohydrate, the new oral rehydration composition can additionally act as a carrier or oral delivery system for other compounds, such as vaccines, drugs, amino acids, vitamins, minerals, nutraceuticals, prebiotics and probiotics, and cause their metabolic uptake to be significantly increased. In prior art, other oral rehydration compositions and solutions rely upon glucose, maltose and or sucrose as an essential part of the carbohydrate base because of its carrying power for the electrolytes, but also for their function as a sweetening agent to improve the organoleptic acceptability of the product. Unfortunately, these sweetening sources also had a high impact on osmolarity. We found that either nutritive or non-nutritive sweeteners may be used in the composition of this invention and still maintain an osmolarity of less than 300 milliosmoles.

The composition of the present invention contains a starch hydrolysate with a DE value between 20 and 30, more preferably DE 25 at levels of greater than 30g/l of solution and more preferably between 35 and 50 g/l of solution. The level and type of carbohydrates and protein of between 0.5% and 1.0% enabled us to increase the carbohydrate levels in the oral rehydration solution for maximum transport, while maintaining low osmolarity. This provides for significant increase in uptake or carrying power for delivery of the vaccines, drugs, amino acids, minerals, vitamins, nutraceuticals, probiotics and /or prebiotics.

We also found that by using the low DE starch hydrolysates described in this invention in making the oral rehydration composition, that we could, if so desired, also additionally use nutritive sweeteners (sucrose, glucose, fructose, or any combination thereof) or non-nutritive sweeteners. Previously, due to the high osmloarity contribution caused by the colloidal starch hydrolysates of Mitchell with a protein content of 0.5% to 1.0% and having a DE of 35 to 50, only functionally insignificant amounts of other simple sugars could be utilized in the oral rehydration composition without increasing the osmolarity of the prepared solution beyond the desired limit of 300 milliosmoles. In the present invention, the lower DE value colloidal starch hydrolysates of Mitchell with a protein content of 0.5% to 1.0%, now make it possible to add a wide range of flavoring, sweeteners, and adjunct agents, to the composition and still remain below the critical 300 milliosmoles for the prepared solution.

The lower DE value increases absorption, and enables us to use as much as 40 to 50 grams of the colloidal starch hydrolysate of Mitchell with a protein content of 0.5% and 1.0%, additionally being able to add flavors, nutritive or non-nutritive sweeteners to deliver the electrolytes, and optionally being able to add vaccines, drugs, vitamins, amino acids, minerals, nutraceuticals, probiotics, prebiotics, or other compositionally functional adjunct agents, while further enhancing the quick delivery and effectiveness of the agent being delivered. The oral rehydration composition still has preferably less than 300 milliosmoles upon dilution to the ready to drink oral rehydration solution. While it is preferable to have less than 300 milliosmoles for rehydration solutions, it may be suitable to have up to 400 milliosmoles for those rehydration solutions that are intended to be utilized on a one time basis, for example, in the case of delivering vaccines. Rehydration solutions, which are intended for continuous use or are expected to be consumed on a regular basis, such as to inhibit or diminish diarrhea, or for purposes of sweat replacement, preferably are less than 300 milliosmoles.

The oral rehydration compositions and systems as described above may be further dried to less than 5% moisture, or concentrated to the equivalent of between 78 and 85 Brix, to achieve a format that is microbiologically stable and suitable for packaging without additional processing. Drying to less than 5% moisture may be done by spray, freeze, or drum drying or other suitable drying method that can achieve a moisture content of less than 5%. Concentration of the rehydration composition may be achieved by the use of a standard evaporator capable of handling viscous fluids and viscosities in excess of 10 Pa·s (100 poise) at 37.8 °C (100 degrees Fahrenheit). These concentrated or dried rehydration compositions and systems, may also be diluted in water to achieve a ready to drink product having a total soluble solids of between of 4.5% to 6.5% and, if desired, further processed in an aseptic system to achieve commercial sterility in any suitable aseptic packaging format.

It should be noted that the term "osmolality", which refers to moles per kilogram, and the term "osmolarity", which specifies moles per liter, while different terms, are being used in this patent interchangeably due to the low density values and hence low impact on the conversion of osmolaity to osmolarity of the oral rehydration solutions.

Below are illustrative formulations of the composition according to the present invention.

**Formula 1**

| **Ingredient** | **Grams/Liter** |
|---|---|
| Water | 953.400 |
| Rice Syrup Solids, 30 DE | 40.000 |
| Sucrose | 20.300 |
| Sodium Chloride | 0.800 |
| Potassium Chloride | 0.300 |
| Trisodium Citrate | 0.600 |
| Lemon Flavor | 0.600 |
| **TOTAL** | **1,016.000** **Osmolarity: 210 mmol/kg** |

**Formula 2**

| **Ingredient** | **Grams/Liter** |
|---|---|
| Water | 956.445 |
| Rice Syrup Solids, 20 DE | 50.000 |
| Sucralose | 0.035 |
| Sodium Chloride | 0.800 |
| Potassium Chloride | 0.400 |
| Citric Acid | 1.300 |
| Trisodium Citrate | 0.600 |
| Mixed Berry Flavor | 0.400 |
| FD&C Red No. 40 | 0.020 |
| **TOTAL** | **1,010.000** **Osmolarity: 170 mmol/kg** |

**Formula 3**

| **Ingredient** | **Grams/Liter** |
|---|---|
| Water | 956.350 |
| Rice Syrup Solids, 25 DE | 40.000 |
| Sucrose | 16.000 |
| Sodium Chloride | 1.150 |
| Potassium Chloride | 1.500 |
| Trisodium Citrate | 3.000 |
| Inulin | 2.000 |
| *Lactobacillus reuteri** | 5x10⁸ CFU |
| *In dry mix form only | |
| **TOTAL** | **1,020.000** **Osmolarity: 290 mmol/kg** |

**Formula 4**

| **Ingredient** | **Grams/Liter** |
|---|---|
| Water | 965.965 |
| Rice Syrup Solids, 25 DE | 45.000 |
| Sucralose | 0.035 |
| Sodium Chloride | 2.300 |
| Potassium Chloride | 1.500 |
| Trisodium Citrate | 2.900 |
| Orange Flavor | 1.000 |
| Citric Acid | 1.300 |
| **TOTAL** | **1,020.000** **Osmolarity: 290 mmol/kg** |

**Formula 5**

| **Ingredient** | **Grams/Liter** |
|---|---|
| Water | 965.384 |
| Rice Syrup Solids, 20 DE | 40.000 |
| Sodium Chloride | 2.300 |
| Potassium Chloride | 1.500 |
| Magnesium Chloride | 1.570 |
| Trisodium Citrate | 2.900 |
| Chicken Broth Flavoring | 1.200 |
| Histidine | 0.146 |
| **TOTAL** | **1,015.000** **Osmolarity: 290 mmol/kg** |

**Formula 6**

| **Ingredient** | **Grams/Liter** |
|---|---|
| Water | 950.449 |
| Rice Syrup Solids, 30 DE | 45.000 |
| Sucralose | 0.050 |
| Sodium Bicarbonate | 10.000 |
| Trisodium Citrate | 2.500 |
| Mango Flavor | 2.000 |
| DukoralTM Killed Cholera Vaccine* | 0.001 |
| * One dose consists of 1011 killed bacteria in 1 milligram of Cholera B subunit. | |
| **TOTAL** | **1,010.000** **Osmolarity: 375 mmol/kg** |

The first illustrative formula utilizes rice syrup solids with a DE of 30, sucrose as a sweetener, sodium chloride and potassium chloride for electrolytes, and trisodium citrate and lemon flavor for flavoring elements. This formulation provides a solution with a total osmolarity of 210 mmol/kg, well under the 400 mmol/kg that causes adverse physiological effects.

The second illustrative formulation utilizes rice syrup solids with a DE of 20, sucralose as a sweetener, sodium chloride and potassium chloride for electrolytes, citric acid, trisodium citrate and mixed berry flavor for flavoring elements and finally, FD&C Red No. 40 for a coloring element. This formulation provides a solution having an osmolarity of 170 mmol/kg.

The third illustrative formulation utilizes rice syrup solids with a DE of 25, sucrose as a sweetener, sodium chloride and potassium chloride for electrolytes, trisodium citrate for a flavoring element and finally, Inulin and *Lactobacillus reuteri* as active ingredients. This formulation provides a solution having an osmolarity of 290 mmol/kg.

The fourth illustrative formulation utilizes rice syrup solids with a DE of 25, sucralose as a sweetener, sodium chloride and potassium chloride for electrolytes, trisodium citrate and orange flavor for flavoring elements and finally, citric acid for an active element. This formulation provides a solution having an osmolarity of 290 mmol/kg.

The fifth illustrative formulation utilizes rice syrup solids with a DE of 20, sodium chloride, potassium chloride and magnesium chloride for electrolytes, trisodium citrate and chicken broth flavoring for flavoring elements and finally, histidine for an active element. This formulation provides a solution having an osmolarity of 290 mmol/kg.

The sixth illustrative formulation utilizes rice syrup solids with a DE of 30, sucralose as a sweetener, sodium bicarbonate for electrolytes, trisodium citrate and mango flavoring for flavoring elements and finally, DukoralTM Killed Cholera Vaccine for as an active element. This formulation provides a solution having an osmolarity of 375 mmol/kg.

The preferred embodiment of the invention is described above in the Description of Preferred Embodiments. While these descriptions directly describe the above embodiments, it is understood that those skilled in the art may conceive modifications and/or variations to the specific embodiments shown and described herein. Any such modifications or variations that fall within the scope of the claims are intended to be included therein as well. Unless specifically noted, it is the intention of the inventor that the words and phrases in the specification and claims be given the ordinary and accustomed meanings to those of ordinary skill in the applicable art(s). The foregoing description of a preferred embodiment and best mode of the invention known to the applicant at the time of filing the application has been presented and is intended for the purposes of illustration and description. It is not intended to be exhaustive or to limit the invention to the precise form disclosed, and many modifications and variations are possible in the light of the above teachings within the scope of the claims. The embodiment was chosen and described in order to best explain the principles of the invention and its practical application and to enable others skilled in the art to best utilize the invention in various embodiments and with various modifications within the scope of the claims as are suited to the particular use contemplated.

## Claims

1. An oral rehydration composition, comprising an effective amount of electrolytes and carbohydrates that are suitable for dissolving in a water base so as to produce an oral rehydration solution having an osmolality of less than 400 milliosmoles; said carbohydrates further comprising a low fiber colloidal hydrolyzed rice suspension comprising, on a dry weight basis, less than 0.1 % fiber, between 0.5% and 1.0% protein, between 0.5% and 1.0% fat, a dextrose equivalency of between 20 and 30, and a glucose and maltose content of less than 20%.

2. The oral rehydration composition according to claim 1 further comprising sufficient water to bring the concentration of the low fiber colloidal hydrolyzed rice suspension to between 30 and 50 grams per liter of solution.

3. The oral rehydration composition according to claim 1 comprising less than 5% water.

4. The oral rehydration composition according to claim 1 concentrated to a syrup of between 78 and 85 Brix.

5. The oral rehydration composition according to claim 1 further comprising an effective amount of sweeteners to improve organoleptic qualities.

6. The oral rehydration composition according to claim 3 or claim 4 or claim 5 further comprising sufficient water to yield a 400 or less milliosmole solution.

7. The oral rehydration composition according to claim 1 further comprising at least one of at least one nutraceutical or at least one functional ingredient.

8. The oral rehydration composition according to claim 7 further comprising water.

9. The oral rehydration composition according to claim 4 or claim 7 or claim 8 comprising less than 5% water.

10. The oral rehydration compositions according to any of claims 1, 5 and 7 further comprising an additional flavour component.

11. The oral dehydration compositions according to any of claims 1, 5 and 7 further comprising an additional acid component for the purpose of pH adjustment.

12. An oral rehydration composition according to claim 2, further comprising a vaccine.

13. Dissolution in a water base of an oral rehydration composition as claimed in any preceding claim for the manufacture of a composition for treating diarrhoea.

14. Dissolution in a water base of an oral rehydration composition as claimed in any preceding claim for the manufacture of a composition for sweat replacement.

## Patentansprüche

1. Orale Rehydrationszusammensetzung, welche eine wirksame Menge von Elektrolyten und Kohlenhydraten aufweist, die zum Auflösen auf Wasserbasis geeignet ist, um so eine orale Rehydrationslösung zu erzeugen, die eine Osmolalität besitzt, die kleiner ist als 400 Milliosmol; wobei die Kohlenhydrate ferner eine hydrolysierte kolloidale Reis-Suspension mit geringem Faseranteil auf Trockenbasis mit weniger als 0,1 Prozent Faser aufweisen, zwischen 0,5 Prozent und 1,0 Prozent Protein, zwischen 0,5 Prozent und 1,0 Prozent Fett, ein Dextrose-Äquivalent zwischen 20 und 30 sowie einen Gehalt an Glucose und Maltose von weniger als 20 Prozent.

2. Orale Rehydrationszusammensetzung nach Anspruch 1, ferner aufweisend ausreichend Wasser, um die Konzentration der hydrolysierten kolloidalen Reis-Suspension mit geringem Faseranteil auf zwischen 30 und 50 Gramm pro Liter Lösung zu bringen.

3. Orale Rehydrationszusammensetzung nach Anspruch 1, die weniger als 5 Prozent Wasser aufweist.

4. Orale Rehydrationszusammensetzung nach Anspruch 1, die zu einem Sirup zwischen 78 und 85 Brix-Grad konzentriert ist.

5. Orale Rehydrationszusammensetzung nach Anspruch 1, ferner aufweisend eine wirksame Menge von Süßungsmitteln, um die organoleptischen Eigenschaften zu verbessern.

6. Orale Rehydrationszusammensetzung nach Anspruch 3 oder Anspruch 4 oder Anspruch 5, ferner aufweisend ausreichend Wasser, um eine Lösung von 400 oder weniger Milliosmol zu liefern.

7. Orale Rehydrationszusammensetzung nach Anspruch 1, ferner aufweisend mindestens ein von mindestens einem nutrazeutischen oder mindestens einem funktionellen Inhaltsstoff.

8. Orale Rehydrationszusammensetzung nach Anspruch 7, ferner aufweisend Wasser.

9. Orale Rehydrationszusammensetzung nach Anspruch 4 oder Anspruch 7 oder Anspruch 8, aufweisend weniger als 5 Prozent Wasser.

10. Orale Rehydrationszusammensetzung nach einem der Ansprüche 1, 5 und 7, ferner aufweisend eine zusätzliche Geschmackskomponente.

11. Orale Rehydrationszusammensetzung nach einem der Ansprüche 1, 5 und 7, ferner aufweisend eine zusätzliche Säurekomponente zum Zwecke der Einstellung des pH-Werts.

12. Orale Rehydrationszusammensetzung nach Anspruch 2, ferner aufweisend einen Impfstoff.

13. Auf Wasser basierende Auflösung einer oralen Rehydrationszusammensetzung nach einem der vorgenannten Ansprüche für die Herstellung einer Zusammensetzung zum Behandeln von Diarrhöe.

14. Auf Wasser basierende Auflösung einer oralen Rehydrationszusammensetzung nach einem der vorgenannten Ansprüche für die Herstellung einer Zusammensetzung zur Schweißsubstitution.

## Revendications

1. Composition orale de réhydratation, comprenant une quantité efficace d'électrolytes et de glucides qui sont appropriés pour se dissoudre dans une base d'eau afin de produire une solution orale de réhydratation présentant une osmolalité inférieure à 400 milliosmoles ; lesdits glucides comprenant en outre une suspension colloïdale de riz hydrolysé à faible teneur en fibre comprenant, sur une base de poids sec, moins de 0,1 % de fibres, entre 0,5 % et 1,0 % de protéines, entre 0,5 % et 1,0 % de matières grasses, un équivalent en dextrose entre 20 et 30 et une teneur en glucose et en maltose inférieure à 20 %.

2. Composition orale de réhydratation selon la revendication 1, comprenant en outre suffisamment d'eau pour porter la concentration de la suspension colloïdale de riz hydrolysé à faible teneur en fibre entre 30 et 50 grammes par litre de solution.

3. Composition orale de réhydratation selon la revendication 1, comprenant moins de 5 % d'eau.

4. Composition orale de réhydratation selon la revendication 1, concentrée pour donner un sirop présentant un Brix entre 78 et 85.

5. Composition orale de réhydratation selon la revendication 1, comprenant en outre une quantité efficace d'édulcorants pour améliorer les qualités organoleptiques.

6. Composition orale de réhydratation selon la revendication 3 ou la revendication 4 ou la revendication 5, comprenant en outre suffisamment d'eau pour donner une solution à 400 milliosmoles ou moins.

7. Composition orale de réhydratation selon la revendication 1, comprenant en outre au moins un nutraceutique ou au moins un ingrédient fonctionnel.

8. Composition orale de réhydratation selon la revendication 7, comprenant en outre de l'eau.

9. Composition orale de réhydratation selon la revendication 4 ou la revendication 7 ou la revendication 8, comprenant moins de 5% d'eau.

10. Compositions orales de réhydratation selon l'une quelconque des revendications 1, 5 et 7, comprenant en outre un composant aromatisant supplémentaire.

11. Compositions orales de réhydratation selon l'une quelconque des revendications 1, 5 et 7, comprenant en outre un composant acide supplémentaire pour ajuster le pH.

12. Composition orale de réhydratation selon la revendication 2, comprenant en outre un vaccin.

13. Dissolution dans une base d'eau d'une composition orale de réhydratation selon l'une quelconque des revendications précédentes, pour la fabrication d'une composition destinée au traitement des diarrhées.

14. Dissolution dans une base d'eau d'une composition orale de réhydratation selon l'une quelconque des revendications précédentes, pour la fabrication d'une composition destinée au remplacement de la transpiration.
